# EUROPÄISCHE PATENTSCHRIFT

(11) **EP 3 247 283 B1**
(45) Veröffentlichungstag und Bekanntmachung des Hinweises auf die Patenterteilung: **06.03.2019**
(21) Anmeldenummer: 16711767.0
(22) Anmeldetag: 20.01.2016
(51) Int. Cl.: A61B 17/00, A61M 25/09

(54) **VORRICHTUNG ZUM FESTKLEMMEN EINES MEDIZINISCHEN FÜHRUNGSDRAHTES**
DEVICE FOR FIRMLY CLAMPING A MEDICAL GUIDE WIRE
DISPOSITIF SERVANT À BLOQUER UN FIL-GUIDE MÉDICAL

(30) Priorität: 21.01.2015 DE 202015000456 U
(43) Veröffentlichungstag der Anmeldung: 29.11.2017
(73) Patentinhaber: Urotech GmbH, 83101 Rohrdorf-Achenmühle (DE)
(72) Erfinder: SCHWARZ, Werner, 83324 Ruhpolding (DE)
(74) Vertreter: Hofstetter, Schurack & Partner
(86) Internationale Anmeldenummer: PCT/DE2016/000020
(87) Internationale Veröffentlichungsnummer: WO 2016/116093

(56) Entgegenhaltungen:
- WO-A1-2006/124580
- WO-A2-2013/052906
- US-A- 4 726 369
- US-A- 6 033 414
- US-A1- 2005 240 120
- US-A1- 2007 219 467

## Beschreibung

Die Erfindung bezieht sich auf eine Vorrichtung zum Festklemmen eines medizinischen Führungsdrahtes, umfassend einen Aufnahmekörper mit einer diesen durchziehenden Längsöffnung für die Aufnahme des Führungsdrahtes, der in der Längsöffnung verschiebbar und verdrehbar ist, und einen Klemmmechanismus, durch dessen Wirksamwerden die Verschieb- und/oder Verdrehbarkeit des in der genannten Längsöffnung aufgenommenen Führungsdrahtes blockierbar ist.

Vorrichtungen der vorstehenden Art sind bereits bekannt; sie werden auch als Torquer bezeichnet. Bei einem derartigen bekannten Torquer (US 5,325,868) ist die Längsöffnung des Aufnahmekörpers mit einander gegenüber liegenden Greifelementen versehen, zwischen denen ein Führungsdraht festgeklemmt werden kann. Die einen Greifelemente sind stationär angeordnet, und die anderen Greifelemente sind beweglich und werden mittels Druckfedern gegen die stationär angeordneten Greifelemente gedrückt. Mittels einer mit den beweglichen Greifelementen verbundenen Taste können diese Greifelemente in einer Einhandbedienung von den stationär angeordneten Greifelementen so gelöst werden, dass ein Führungsdraht zwischen die betreffenden Greifelemente hindurch geführt werden kann. Wird die Taste anschließend wieder losgelassen, so wird der Führungsdraht durch die Greifelemente festgeklemmt. Obwohl hierdurch ein sicheres Festklemmen des Führungsdrahtes ermöglicht ist, besteht jedoch zuweilen der Wunsch, ein solches Festklemmen eines Führungsdrahtes mit einem geringeren konstruktiven Aufwand zu erreichen.

Bei einem anderen bekannten Torquer (US 6,030,349) ist die Längsöffnung des Aufnahmekörpers durch einen seitlich offenen Längsschlitz gebildet, und der Klemmmechanismus ist durch ein Tastenteil ebenfalls mit einem seitlich offenen Längsschlitz gebildet. Dieses Tastenteil wird durch Elastomerkörper in einer solchen Stellung gehalten, dass dessen Längsschlitz und der Längsschlitz des Aufnahmekörpers gegeneinander versetzt sind. Wird jedoch das Tastenteil in den Aufnahmekörper hinein gedrückt, so fluchten die beiden seitlich offenen Längsschlitze derart, dass von ihnen ein medizinischer Führungsdraht aufgenommen werden kann. Nach Loslassen des Tastenteiles wird dann der betreffende Führungsdraht in den beiden Längsschlitzen festgeklemmt. Obwohl auch hierdurch ein sicheres Festklemmen des Führungsdrahtes ermöglicht ist, besteht angesichts dieses Torquers jedoch ebenfalls der Wunsch, ein Festklemmen eines Führungsdrahtes mit einem geringeren konstruktiven Aufwand zu erreichen.

Bei einem anderen bekannten Torquer (DE 10 2004 017 734 B4), der ebenfalls einen Ein-Hand-Betrieb ermöglicht, besteht der Aufnahmekörper aus zwei um eine Längsachse schwenkbare Abschnitte, die zum Einbringen eines Führungsdrahtes seitlich zu öffnen sind. Ist der Führungsdraht in diese Abschnitte eingelegt, werden diese zusammengedrückt, wodurch die Bewegung des Führungsdrahts begrenzt wird. Zusätzlich weist dieser bekannte Torquer noch ein Klemmmittel in Form eines an einem der genannten Abschnitte gegen eine Rückstellkraft schwenkbar angeordneten Klemmhebel auf, der in der geschlossenen Stellung mit einem an dem anderen Abschnitt vorgesehenen Widerlager zusammenwirkt. Obwohl auch hierdurch ein sicheres Festklemmen eines Führungsdrahtes ermöglicht ist, besteht jedoch auch hier der Wunsch, ein Festklemmen eines Führungsdrahtes mit einem geringeren konstruktiven Aufwand zu erreichen.

Es ist schließlich auch schon eine Vorrichtung zur Betätigung eines medizinischen Führungs- oder Transportdrahtes bekannt (DE 10 2012 104 961 A1), umfassend ein Gehäuse mit einer Durchführung zur Aufnahme des Führungs- oder Transportdrahtes und wenigstens ein Halteelement, das die Durchführung zumindest abschnittweise begrenzt und durch eine äußere Kraft betätigbar oder selbsthemmend derart ausgebildet ist, dass eine kraftschlüssige Verbindung zwischen dem Halteelement und dem Führungs- oder Transportdrahtes erzeugbar ist. Bei dieser bekannten Vorrichtung ist wenigstens eine Erfassungseinrichtung zum Erfassen von Kräften und/oder Momenten vorgesehen, die bei der Betätigung des Führungs- oder Transportdrahtes auf diesen wirken. Außerdem ist wenigstens eine Rückmeldeeinrichtung vorgesehen, die mit der Erfassungseinrichtung zur Abgabe mindestens eines Signals gekoppelt oder koppelbar ist. Durch das Vorsehen der Erfassungseinrichtung können anhand der erwähnten Kräfte und/oder Momente Daten gewonnen werden, die Rückschlüsse auf die Belastung von Hohlwandgefäßwandungen gezogen werden, in deren Bereich der betreffende Führungs- oder Transportdraht angewandt wird. Angesichts des für ein sicheres Festklemmen eines Führungs- oder Transportdrahtes erforderlichen konstruktiven Aufwands, besteht allerdings auch hier der Wunsch, ein Festklemmen eines Führungsdrahtes mit einem geringeren konstruktiven Aufwand zu erreichen.

US 2005/240120 A1 offenbart eine Vorrichtung zum Festklemmen eines medizinischen Führungsdrahtes, umfassend einen Aufnahmekörper mit einer diesen durchziehenden Längsöffnung für die Aufnahme des Führungsdrahtes der in der Längsöffnung verschiebbar und verdrehbar ist, und einen Klemmmechanismus, durch dessen Wirksamwerden die Verschieb- und/oder Verdrehbarkeit des in der genannten Längsöffnung aufgenommenen Führungsdrahtes blockierbar ist.

Der vorliegenden Erfindung liegt daher die Aufgabe zu Grunde, einen Weg zu zeigen, wie bei einer Vorrichtung der eingangs genannten Art mit einem geringeren konstruktiven Aufwand als bei den bisher bekannten Vorrichtungen zum Festklemmen eines infolge seiner Eigenelastizität federnden medizinischen Führungsdrahtes ausgekommen werden kann.

Gelöst wird die vorstehend aufgezeigte Aufgabe bei einer Vorrichtung der eingangs genannten Art erfindungsgemäß dadurch, dass der Klemmmechanismus entweder nur an einem Ende der Längsöffnung des Aufnahmekörpers oder an jedem Ende der Längsöffnung des Aufnahmekörpers gebildet ist, dass der jeweilige Klemmmechanismus einen an seinem zugehörigen Ende des Aufnahmekörpers quer zu der Längsöffnung des Aufnahmekörpers verlaufenden erweiterten Öffnungsbereich, der sich zu der Längsöffnung hin über eine Strecke erstreckt, die kürzer ist als die Längsöffnung lang ist, und eine Klemmgliedanordnung aufweist,
und dass die jeweilige Klemmgliedanordnung zwischen ihrem erweiterten Öffnungsbereich und der genannten Längsöffnung mit dem Aufnahmekörper elastisch ausbiegbar verbunden ist, sich parallel zu der Längsöffnung über eine Distanz erstreckt, die kürzer ist als die genannte Strecke in Richtung der Längsöffnung lang ist, und an einer von dem betreffenden Ende abgewandten, in dem Öffnungsbereich liegenden Seite einen dem zugehörigen Öffnungsbereich zugewandten Kantenteil aufweist, an dem der Führungsdraht nach seinem Verschwenken aus der Längsöffnung an dem betreffenden Ende des Aufnahmekörpers und an der Klemmgliedanordnung vorbei in den an diesem Ende des Aufnahmekörpers vorhandenen Öffnungsbereich hinein unter Blockierung seiner Verschieb- und/oder Verdrehbarkeit in Anlage bringbar ist

Die Erfindung zeichnet sich durch den Vorteil eines besonders geringen konstruktiven Aufwands aus, um einen infolge seiner Eigenelastizität federnden medizinischen Führungsdraht sicher festzuklemmen. Die gemäß der Erfindung für ein solches Festklemmen maßgebende konstruktive Gestaltung der Vorrichtung umfasst lediglich die Erweiterung der genannten Längsöffnung zu einem Öffnungsbereich, in welchem sich die Klemmgliedanordnung mit einem Kantenteil befindet, an welchem der Führungsdraht nach seinem Verschwenken aus der genannten Längsöffnung in den Öffnungsbereich anliegt und dadurch sicher festgehalten wird. Der damit verbundene konstruktive Aufwand ist deutlich geringer als jener bei den eingangs betrachteten bekannten Vorrichtungen bzw. Torquern. Außerdem zeichnet sich die Vorrichtung gemäß der Erfindung dadurch aus, dass sie eine einfache Einhandbedienung ermöglicht, durch die ein Führungsdraht sowohl in der betreffenden Vorrichtung festgeklemmt als auch aus seinem Festklemmen wieder gelöst werden kann, um in der Vorrichtung ungehindert verschoben werden zu können.

Vorzugsweise verläuft der jeweilige Öffnungsbereich unter einem Winkel zwischen etwa 10° und 120° in Bezug auf die Längsöffnung des Aufnahmekörpers. Eine solche Winkelanordnung hat sich als besonders günstig für das angestrebte Festklemmen eines Führungsdrahtes herausgestellt.

Gemäß einer zweckmäßigen Weiterbildung der Erfindung ist die jeweilige Klemmgliedanordnung zwischen der Längsöffnung und dem Öffnungsbereich durch ein einziges Klemmelement gebildet, welches mit einem Zwischenraum zu einem Randbereich der Längsöffnung vorgesehen ist, und der Zwischenraum weist einen ein Hindurchdrücken des Führungsdrahtes aus der Längsöffnung in den genannten Öffnungsbereich und von diesem in die Längsöffnung ermöglichenden Abstand auf. Hierdurch ergibt sich der Vorteil eines besonders geringen konstruktiven Aufwands für das Festklemmen eines Führungsdrahtes.

Gemäß einer anderen zweckmäßigen Weiterbildung der Erfindung ist die jeweilige Klemmgliedanordnung zwischen der Längsöffnung und ihrem zugehörigen Öffnungsbereich durch zwei Klemmelemente gebildet, die unter Zurücklassen eines Zwischenraumes zwischen ihnen jeweils an diametral gegenüber liegenden Stellen von Verbindungsbereichen zwischen der Längsöffnung und dem betreffenden Öffnungsbereich vorgesehen sind, und der Zwischenraum weist einen ein Hindurchdrücken des Führungsdrahtes aus der Längsöffnung in den genannten Öffnungsbereich und von diesem in die Längsöffnung ermöglichenden Abstand auf. Hierdurch ergibt sich der Vorteil eines besonders geringen konstruktiven Aufwands für das Festklemmen eines Führungsdrahtes.

Zweckmäßigerweise weist das jeweilige Klemmelement auf seiner der Längsöffnung zugewandten Seite eine Abschrägungsfase auf. Dadurch lässt sich der Führungsdraht aus der genannten Längsöffnung des Aufnahmekörpers in den zugehörigen Öffnungsbereich besonders leicht verschwenken.

Vorzugsweise ist das jeweilige Klemmelement an dem Verbindungsbereich zwischen der Längsöffnung und seinem zugehörigen Öffnungsbereich an dem Aufnahmekörper angeformt. Dies bringt den Vorteil mit sich, dass der Aufnahmekörper samt des jeweiligen Klemmelements in einem Formungsvorgang, wie in einem Spritzgießvorgang herzustellen ist.

Gemäß einer anderen zweckmäßigen Ausgestaltung der Erfindung besteht das jeweilige Klemmelement aus einem vom Material des Aufnahmekörpers verschiedenen Material und ist an einem Verbindungsbereich zwischen der Längsöffnung und seinem zugehörigen Öffnungsbereich an dem Aufnahmekörper gesondert befestigt. Hierdurch ergibt sich der Vorteil, dass das jeweilige Klemmelement beispielsweise aus einem Material hergestellt sein kann, welches härter ist als das Material des Aufnahmekörpers. Dadurch lassen sich an dem jeweiligen Klemmelement Kanten erzielen, die für das angestrebte Festklemmen des Führungsdrahtes besonders wirksam sind. Die genannte Befestigung des jeweiligen Klemmelements an dem Verbindungsbereich zwischen der Längsöffnung und seinem zugehörigen Öffnungsbereich kann beispielsweise durch eine Klebverbindung oder durch Laserschweißen erfolgen.

Vorzugsweise besteht die Vorrichtung gemäß der Erfindung aus Kunststoff, insbesondere einem biokompatiblen Kunststoff. Dadurch lässt sich die betreffende Vorrichtung problemlos in Medizinbereichen einsetzen, in denen sie mit Körpern von Individuen und insbesondere mit deren Körperflüssigkeiten in Kontakt kommen kann.

Gemäß einer weiteren zweckmäßigen Ausgestaltung der Erfindung ist der Aufnahmekörper auf seiner Außenfläche zumindest partiell phosphoreszierend oder fluoreszierend. Dies ermöglicht es in vorteilhafter Weise, die Vorrichtung gemäß der Erfindung nach zuvor erfolgter Bestrahlung mit für die Entfaltung der Phosphoreszenz oder Fluoreszenz passender Strahlung zusammen mit dem von ihr aufgenommenen Führungsdraht in üblicher Weise abgedunkelten Arbeitsräumen, wie Operationssälen wieder schnell aufzufinden.

Alternativ oder zusätzlich zu der zuletzt betrachteten zweckmäßigen Ausgestaltung der Erfindung enthält die Vorrichtung gemäß der Erfindung wenigstens eine Lichtquelle, insbesondere in einer in dem aus einem lichtdurchlässigen Material bestehenden Aufnahmekörper gebildeten Aufnahmeöffnung. Auch hierdurch ergibt sich der Vorteil, dass die Vorrichtung gemäß der Erfindung zusammen mit dem von ihr aufgenommenen Führungsdraht in üblicher Weise abgedunkelten Arbeitsräumen, wie Operationssälen wieder schnell aufzufinden ist.

An Hand von Zeichnungen wird die Erfindung nachstehend an einigen Ausführungsbeispielen näher erläutert.

In den Zeichnungen zeigen
- Fig. 1: in nicht maßstäblichem Maßstab eine Perspektivansicht eines ersten Ausführungsbeispiels einer Vorrichtung gemäß der Erfindung mit einem Führungsdraht,
- Fig. 2: eine Längsschnittansicht durch die in Fig. 1 dargestellte Vorrichtung,
- Fig. 3: eine Draufsicht auf das in Fig. 1 auf der rechten Seite liegende Ende der betreffenden Vorrichtung zusammen mit einem Führungsdraht,
- Fig. 4: die gleiche Draufsicht wie Fig. 3, allerdings ohne den Führungsdraht,
- Fig. 5: die in Fig. 1 gezeigte Vorrichtung in einer anderen Perspektivdarstellung ebenfalls in nicht maßstäblichem Maßstab und mit einem Teilausschnitt,
- Fig. 6: eine Schnittansicht der in Fig. 5 dargestellten Vorrichtung längs der dort eingetragenen Schnittlinie I - I,
- Fig. 7: in einer ähnlichen Perspektivdarstellung wie Fig. 5 eine Vorrichtung gemäß einem zweiten Ausführungsbeispiel der Erfindung,
- Fig. 8: in nicht maßstäblichem Maßstab eine Schnittansicht der in Fig. 7 dargestellten Vorrichtung längs der dort eingetragenen Schnittlinie II - II,
- Fig. 9: in nicht maßstäblichem Maßstab eine Schnittansicht einer Vorrichtung gemäß einem dritten Ausführungsbeispiel der Erfindung,
- Fig. 10: in nicht maßstäblichem Maßstab eine Schnittansicht einer Vorrichtung gemäß einem vierten Ausführungsbeispiel der Erfindung und
- Fig. 11: in nicht maßstäblichem Maßstab eine Schnittansicht einer Vorrichtung gemäß einem fünften Ausführungsbeispiel der Erfindung.

Bevor auf die Zeichnungen näher eingegangen wird, sei angemerkt, dass in sämtlichen Figuren gleiche oder einander entsprechende Einrichtungen bzw. Elemente mit gleichen Bezugszeichen bezeichnet sind.

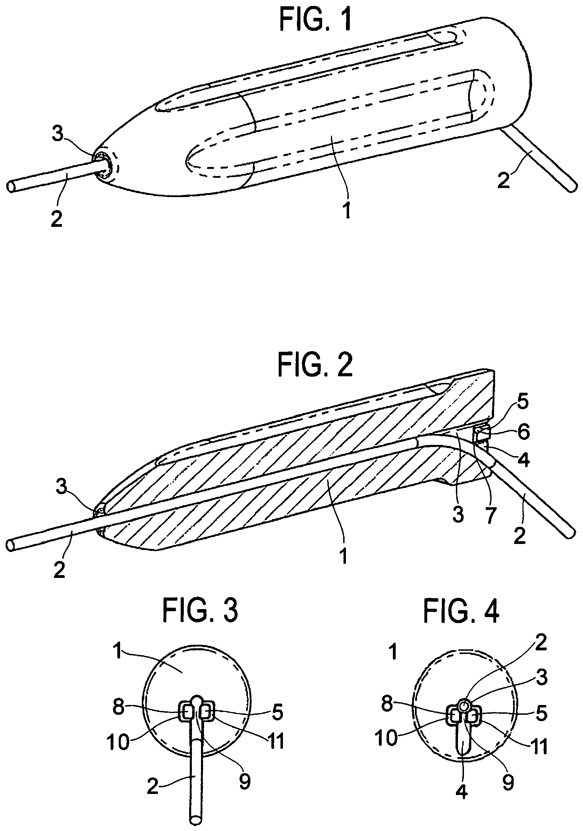

In Fig. 1 ist in einem nicht maßstäblichen Maßstab eine Perspektivansicht eines ersten Ausführungsbeispiels einer Vorrichtung gemäß der Erfindung dargestellt. Die betreffende Vorrichtung weist hier einen zylinderförmigen Aufnahmekörper 1 mit einem runden oder ovalen Querschnitt; der Aufnahmekörper 1 besteht vorzugsweise aus einem biokompatiblen Kunststoff, wie Acrylnitril-Butadien-Styrol (ABS), von dem ein medizinischer Führungsdraht 2 aufgenommen ist, und zwar in einer den Aufnahmekörper 1 in dessen Längsrichtung durchziehenden Längsöffnung 3. Der Durchmesser der Längsöffnung 3 ist dabei so groß, dass in deren Längsrichtung der Führungsdraht 2 ungehindert gleitend verschoben werden kann.

In der Praxis kann der Führungsdraht einen Durchmesser zwischen 0,5 und 1mm besitzen; er besteht üblicherweise aus einem federelastischen Material, wie Edelstahl, und ist mit einem Kunststoffüberzug versehen. Der Aufnahmekörper 1 kann einen Außendurchmesser von beispielsweise 10mm und eine Länge von einigen Zentimetern, wie beispielsweise 5cm besitzen.

An seinem in Fig. 1 links liegenden Ende, welches als distales Ende betrachtet wird, verläuft der Aufnahmekörper 1 von seiner Außenfläche zu seiner Längsöffnung 3 hin konisch zusammen. An seinem in Fig. 1 rechts liegenden Ende, welches als proximales Ende betrachtet wird, weist der Aufnahmekörper 1 eine plane Fläche auf, aus der der Führungsdraht 2 aus der Längsöffnung 3 herausgeschwenkt ist. Durch dieses Herausschwenken wird der Führungsdraht 2, wie noch ersichtlich wird, in dem Aufnahmekörper 1 so festgeklemmt, dass er in Bezug auf den Aufnahmekörper 1 nicht mehr oder nicht mehr leicht verdreh- und/oder verschiebbar ist. Um den zylinderförmigen Umfangsbereich des Aufnahmekörpers 1 herum sind einige Griffmulden vorgesehen, die ein Erfassen und Festhalten des Aufnahmekörpers 1 erleichtern.

In Fig. 2 ist die in Fig. 1 dargestellte Vorrichtung in einer Längsschnittansicht gezeigt. In dieser Schnittansicht erkennt man am rechten proximalen Ende des Aufnahmekörpers 1, dass dort quer zu der Längsöffnung 3 des Aufnahmekörpers 1 ein erweiterter Öffnungsbereich 4 verläuft, der sich über eine Strecke erstreckt, die kürzer ist als die Längsöffnung 3 lang ist. Anders ausgedrückt heißt dies, dass die Längsöffnung 3 des Aufnahmekörpers 1 an dessen proximalen Ende mit dem erweiterten Öffnungsbereich 4 versehen ist. Dieser Öffnungsbereich 4 verläuft hier unter einem Winkel von etwa 45° in Bezug auf die Längsöffnung 3 des Aufnahmekörpers 1. Generell kann dieser Winkel allerdings zwischen etwa 10° und 120° in Bezug auf die Längsöffnung 3 des Aufnahmekörpers 1 betragen. Bei einem Winkel, der größer ist als etwa 45° ist dann der Aufnahmekörper 1 auf der Seite offen, auf der der Führungsdraht 2 aus ihm herausgeschwenkt wird.

In dem am proximalen Ende des Aufnahmekörpers 1 vorgesehenen erweiterten Öffnungsbereich 4 ist in Fig. 2 ein quaderförmiges Element dargestellt, welches für den Führungsdraht 2 ein Klemmelement 5 darstellt. Dieses zu einer noch zu betrachtenden Klemmgliedanordnung gehörende Klemmelement 5 ist mit seiner von dem proximalen Ende des Aufnahmekörpers 1 abgewandten Seite mit dem Aufnahmekörper 1 elastisch ausbiegbar verbunden. Diese elastische Ausbiegbarkeit wird gemäß der Erfindung genutzt, um den zunächst in der Längsöffnung 3 ungehindert verschiebbaren Führungsdraht 2 an dem proximalen Ende des Aufnahmekörpers 1 aus der Längsöffnung in den erweiterten Öffnungsbereich 4 hinein zu verschwenken. Auf seiner der Längsöffnung 3 des Aufnahmekörpers 1 zugewandten Seite weist das Klemmelement 5 eine Abschrägungsfase 6 auf, auf deren Bedeutung weiter unten noch näher eingegangen wird.

Das erwähnte Klemmelement 5 erstreckt sich parallel zu der Längsöffnung 3 über eine Distanz, die kürzer ist als die oben genannte Strecke des erweiterten Öffnungsbereichs 4 in Richtung der Längsöffnung 3 lang ist. Genauer gesagt erstreckt sich das Klemmelement 5 vom proximalen Ende des Aufnahmekörpers 1 soweit in diesen hinein, dass der Führungsdraht 2 nach seinem Verschwenken aus der Längsöffnung 3 an dem betreffenden Ende in den erweiterten Öffnungsbereich 4 zwischen einer dem proximalen Ende des Aufnahmekörpers 1 zugewandten Seite und einem Kantenteil 7 des Klemmelements 5 anzuliegen vermag, welches das Klemmelement 5 zwischen seiner von dem betreffenden proximalen Ende abgewandten, in dem erweiterten Öffnungsbereich 4 liegenden Seite und seiner von der Längsöffnung 3 abgewandten Seite aufweist.

An diesem Kantenteil 7 des Klemmelements 5 lässt sich der Führungsdraht 2 nach seinem Verschwenken aus der Längsöffnung 3 an dem proximalen Ende des Aufnahmekörpers 1 heraus und an dem Klemmelement 5 der Klemmgliedanordnung vorbei in den erwähnten erweiterten Öffnungsbereich 4 hinein bringen und ist durch seine Anlage an dem genannten Kantenteil 7 in seiner Verdrehbarkeit und in seiner Verschiebbarkeit zumindest in Richtung zum proximalen Ende des Aufnahmekörpers 1 hin blockiert. Dabei erleichtert die an dem Klemmelement 5 vorgesehene Abschrägungsfase 6 das Verschwenken des Führungsdrahtes 2 aus der Längsöffnung 3 des Aufnahmekörpers 1. Soll die beschriebene Blockierung des Führungsdrahtes 2 wieder aufgehoben werden, so wird dieser am proximalen Ende des Aufnahmekörpers 1 wieder zu der Längsöffnung 3 zurück geschwenkt.

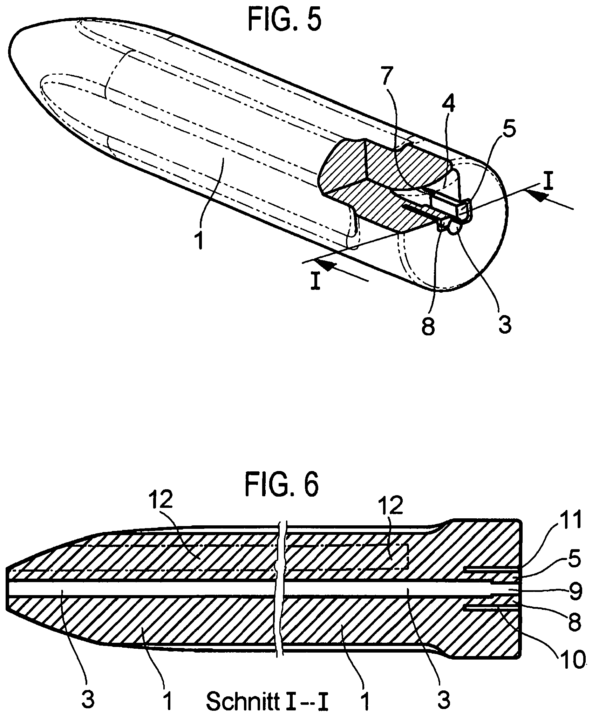

In Fig. 3 und 4 sind Draufsichten auf das proximale Ende des Aufnahmekörpers 1 und damit der in Fig. 1 dargestellten Vorrichtung insgesamt gezeigt. Dabei zeigt Fig. 3 die Draufsicht mit in seiner Klemmposition befindlichem Führungsdraht 2, und Fig. 4 zeigt die Draufsicht mit aus seiner Klemmposition gelöstem Führungsdraht 2. In beiden Draufsichten sieht man neben dem Klemmelement 5 noch ein weiteres Klemmelement 8, welches zusammen mit dem Klemmelement 5 die oben erwähnte Klemmgliedanordnung bildet. Das Klemmelement 8 ist in dem Aufnahmekörper 1 diametral gegenüber dem Klemmelement 5 in einem gewissen Abstand von diesem - also mit einem Zwischenraum 9 von diesem - positioniert. Im Übrigen ist das Klemmelement 8 in gleicher Weise gestaltet wie das Klemmelement 5, also auch mit einer der Abschrägungsfase 6 entsprechenden Abschrägungsfase und mit einem dem Kantenteil 7 entsprechenden Kantenteil. Beide Klemmelemente 5 und 8 sind hier als abstehende Elemente in dem Aufnahmekörper 1 zusammen mit diesem gebildet. Es ist aber auch möglich, die beiden Klemmelemente 5 und 8 als gesonderte Elemente und aus einem anderen, vorzugsweise biokompatiblen Material, beispielsweise aus Polyoxymethylen (POM) vorzusehen als aus dem Material, aus dem der Aufnahmekörper 1 besteht. Diese gesonderten Klemmelemente sind mit dem Aufnahmekörper 1 an den Stellen zu verbinden, beispielsweise durch Ankleben oder Anschweißen, an denen die Klemmelemente 5 und 8 gemäß Fig. 5 und 6 vorgesehen sind.

Die vorstehend erwähnte Klemmgliedanordnung mit den beiden Klemmelementen 5 und 8 blockiert gemäß Fig. 3 den Führungsdraht 2 nach seinem Herausschwenken aus der Längsöffnung 3 am proximalen Ende des Aufnahmekörpers 1 in seiner Verdrehbarkeit und in seiner Verschiebbarkeit zumindest in Richtung zum proximalen Ende des Aufnahmekörpers 1 hin. Nach seinem Zurückschwenken in die Längsöffnung 3 gemäß Fig. 4 ist die Blockierung des Führungsdrahtes 2 wieder aufgehoben. Das Klemmelement 8 ist in dem Aufnahmekörper 1 diametral gegenüber dem Klemmelement 5 in einem gewissen Abstand von diesem - also mit einem Zwischenraum 9 von diesem - positioniert. Auf ihren Seiten, welche von dem genannten Zwischenraum 9 abgewandt sind, sind zwischen den beiden Klemmelementen 5 und 8 und Innenwandbereichen des Aufnahmekörpers 1 Zwischenräume 10 bzw. 11 vorgesehen. Die Längsabmessungen der Zwischenräume 10 und 11 in der Längsrichtung des Aufnahmekörpers 1 sind dabei größer als wie die Länge jedes der Klemmelemente 5 und 8 in der betreffenden Längsrichtung. Vorzugsweise sind die Längsabmessungen der Zwischenräume 10 und 11 in der Längsrichtung des Aufnahmekörpers 1 mindestens etwa doppelt so groß wie die gerade erwähnte Länge jedes der Klemmelemente 5 und 8.

In Fig. 5 und Fig. 6 sind die vorstehend erläuterten Verhältnisse am proximalen Ende des Aufnahmekörpers 1 noch näher spezifiziert. Dabei zeigt Fig. 5 die in Fig. 1 gezeigte Vorrichtung in einer anderen Perspektivdarstellung ebenfalls in nicht maßstäblichem Maßstab, allerdings mit einem Teilausschnitt, und Fig. 6 zeigt eine Schnittansicht der in Fig. 5 dargestellten Vorrichtung längs der dort eingetragenen Schnittlinie I - I. Aus beiden Fig. 5 und 6 ist näher ersichtlich, wie die Klemmgliedanordnung mit den beiden Klemmelementen 5 und 8 am proximalen Ende des Aufnahmekörpers 1 ausgebildet ist. Dabei ist insbesondere aus Fig. 6 ersichtlich, dass die beiden Klemmelemente 5 und 8 zur Längsmitte des Aufnahmekörpers hin verlaufende Auskragungen aufweisen. Die Abmessungen dieser Auskragungen in Richtung zu der Längsmitte des Aufnahmekörpers 1 entsprechen jeweils der Abmessung der Zwischenräume 10 bzw. 11 in der diametralen Querrichtung des Aufnahmekörpers 1; sie sind vorzugsweise zumindest gleich den betreffenden Querabmessungen dieser Zwischenräume 10 bzw. 11 in der diametralen Querrichtung, also den Abmessungen, die aus den Draufsichten gemäß Fig. 3 und 4 und aus der Schnittansicht gemäß Fig. 6 in der diametralen Querrichtung des Aufnahmekörpers 1 vorhanden sind.

Die Abmessungen der Zwischenräume 9, 10 und 11 sind somit so gewählt, dass die beiden Klemmelemente 5 und 8 bei einem Verschwenken des Führungsdrahtes 2 zwischen dessen Klemmposition und dessen Nicht-Klemmposition mit ihren von dem Zwischenraum 9 abgewandten Seiten in den Zwischenräumen 10 bzw. 11 aufgenommen sind und dass der Führungsdraht 2 problemlos durch den Zwischenraum 9 hindurch geschwenkt werden kann.

Zusätzlich ist in Fig. 6 innerhalb des Aufnahmekörpers 1 noch ein durch Strichpunktlinien markierten Bereich angegeben, der eine Aufnahmeöffnung 12 darstellen soll, auf deren Bedeutung weiter unten noch eingegangen wird. Diese Aufnahmeöffnung 12 erstreckt sich hier vom distalen Ende des Aufnahmekörpers 1 nur über einen Teil der Gesamtlänge des Aufnahmekörpers 1; sie kann aber quer zur Längsrichtung des Aufnahmekörpers 1 in diesem vorgesehen sein.

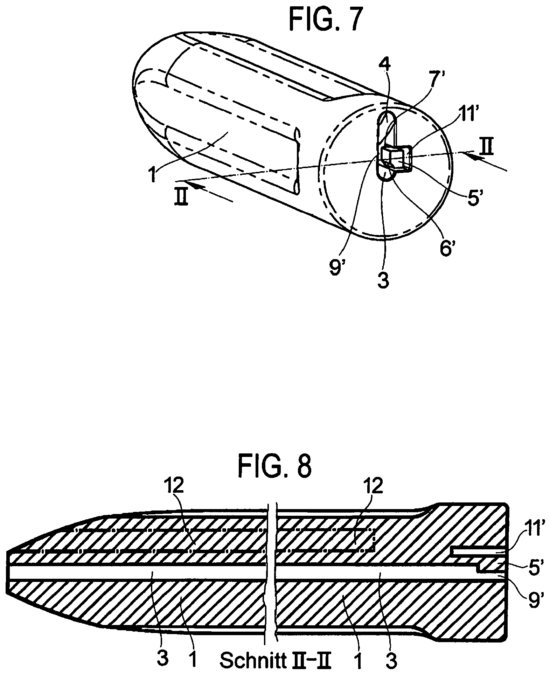

Während zuvor an Hand der Fig. 1 bis 6 ein erstes Ausführungsbeispiel der Vorrichtung gemäß der Erfindung beschrieben worden ist, bei dem die Klemmgliedanordnung zwei Klemmelemente, nämlich die Klemmelemente 5 und 8 enthält, zeigen die Fig. 7 und Fig. 8 eine Vorrichtung gemäß einem zweiten Ausführungsbeispiel der Erfindung, bei dem die Klemmgliedanordnung lediglich ein Klemmelement 5' enthält. Dabei zeigt Fig. 7 in einer ähnlichen Perspektivdarstellung wie Fig. 5 diese Vorrichtung gemäß dem zweiten Ausführungsbeispiel der Erfindung, und Fig. 8 zeigt in nicht maßstäblicher Darstellung eine Schnittansicht der in Fig. 7 dargestellten Vorrichtung längs der dort eingetragenen Schnittlinie II - II. Der in Fig. 7 und 8 dargestellte Aufnahmekörper 1 ist ebenfalls zylinderförmig ausgebildet und er weist ebenfalls einen runden oder ovalen Querschnitt auf.

Das in der in Fig. 7 und 8 dargestellten Vorrichtung vorgesehene Klemmelement 5' entspricht weitgehend dem Klemmelement 5 bei der Vorrichtung gemäß dem ersten Ausführungsbeispiel der Erfindung. Das Klemmelement 5' weist demgemäß eine Abschrägungsfase 6' und einen Kantenteil 7' auf. Allerdings ist hier die Auskragung des Klemmelements 5' in Richtung quer zu der Längsmitte des Aufnahmekörpers 1 größer als und vorzugsweise doppelt so groß wie jene des Klemmelements 5. Außerdem sind hier nur zwei Zwischenräume 9' und 11' vorgesehen.

Die Abmessung der Auskragung des Klemmelements 5' in Richtung zur Längsmitte des Aufnahmekörpers 1 entspricht der Abmessung des Zwischenraumes 11' in der diametralen Querrichtung des Aufnahmekörpers 1; sie ist vorzugsweise zumindest gleich der betreffenden Querabmessung dieses Zwischenraumes 11' in der diametralen Querrichtung, also der Abmessung, die aus der Schnittansicht gemäß Fig. 8 in der diametralen Querrichtung des Aufnahmekörpers 1 vorhanden ist.

Die Längsabmessung des Zwischenraumes 11' in der Längsrichtung des Aufnahmekörpers 1 ist dabei größer als die Länge des Klemmelements 5' in der betreffenden Längsrichtung. Vorzugsweise ist die Längsabmessung des Zwischenraumes 11' in der Längsrichtung des Aufnahmekörpers 1 mindestens etwa doppelt so groß wie die gerade erwähnte Länge des Klemmelements 5'.

Die Abmessung des Zwischenraumes 9' ist zusammen mit den Abmessungen des Zwischenraumes 11' so gewählt, dass das einzige Klemmelement 5' bei einem Verschwenken des Führungsdrahtes 2 zwischen dessen Klemmposition und dessen Nicht-Klemmposition mit seiner von dem Zwischenraum 9' abgewandten Seite in dem Zwischenraum 11' aufgenommen wird und dass der Führungsdraht 2 problemlos durch den Zwischenraum 9' hindurch geschwenkt werden kann.

In Fig. 8 ist innerhalb des Aufnahmekörpers 1 ebenfalls ein durch Strichpunktlinien markierten Bereich angegeben, der eine Aufnahmeöffnung 12 darstellen soll, auf deren Bedeutung weiter unten noch eingegangen wird. Diese Aufnahmeöffnung 12 erstreckt sich auch hier vom distalen Ende des Aufnahmekörpers 1 nur über einen Teil der Gesamtlänge des Aufnahmekörpers 1; sie kann aber auch hier quer zur Längsrichtung des Aufnahmekörpers 1 in diesem vorgesehen sein.

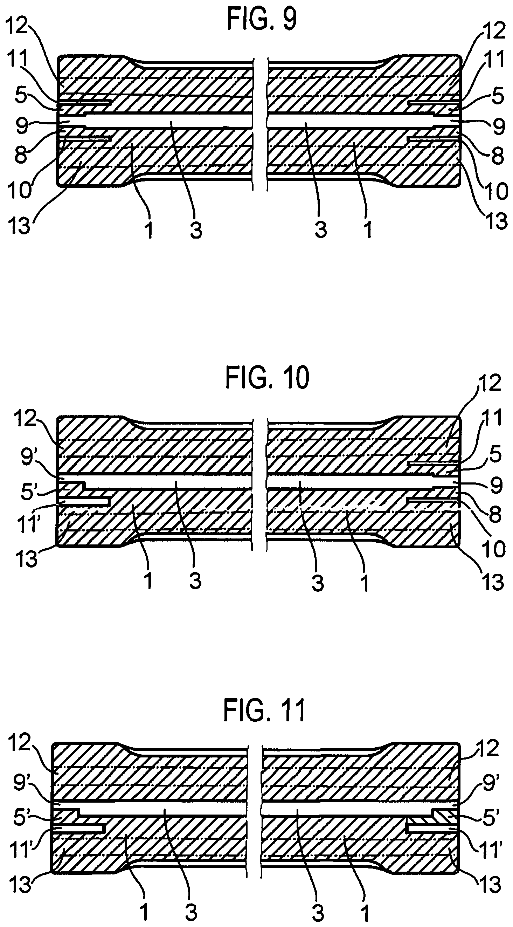

Vorstehend sind zwei Ausführungsbeispiele der Vorrichtung gemäß der Erfindung beschrieben worden, bei denen die Klemmgliedanordnung lediglich am proximalen Ende des Aufnahmekörpers 1 und damit der gesamten Vorrichtung vorgesehen ist. In Fig. 9 bis 11 sind drei weitere Ausführungsbeispiele der Vorrichtung gemäß der Erfindung dargestellt, bei denen Klemmgliedanordnungen sowohl am proximalen Ende als auch am distalen Ende des Aufnahmekörpers 1 und somit der gesamten Vorrichtung vorgesehen sind. Durch diese Klemmgliedanordnungen lässt sich die Klemmwirkung auf den Führungsdraht 2 in dem jeweiligen Aufnahmekörper 1 noch weiter steigern, so dass er weder vom distalen Ende noch vom proximalen Ende des Haltekörpers 1 aus diesem herausgezogen werden kann. Sämtliche Vorrichtungen gemäß Fig. 9, 10 und 11 weisen ebenfalls einen zylinderförmigen Aufnahmekörper 1 mit einem runden oder ovalen Querschnitt auf.

So zeigt Fig. 9 in nicht maßstäblichem Maßstab eine Schnittansicht einer Vorrichtung gemäß einem dritten Ausführungsbeispiel der Erfindung, bei der zwei Klemmgliedanordnungen vorhanden sind, wie sie in der Schnittansicht gemäß Fig. 6 zu sehen ist. Fig. 10 zeigt in nicht maßstäblichem Maßstab eine Schnittansicht einer Vorrichtung gemäß einem vierten Ausführungsbeispiel der Erfindung, bei der zwei verschiedene Klemmgliedanordnungen vorgesehen sind, nämlich eine Klemmgliedanordnung, wie sie in der Schnittansicht gemäß Fig. 6 zu sehen ist, und eine Klemmgliedanordnung, wie sie in der Schnittansicht gemäß Fig. 8 zu sehen ist. Fig. 11 zeigt schließlich in nicht maßstäblichem Maßstab eine Schnittansicht einer Vorrichtung gemäß einem fünften Ausführungsbeispiel der Erfindung, bei der zwei Klemmgliedanordnungen vorhanden sind, wie sie in der Schnittansicht gemäß Fig. 8 zu sehen ist. Für alle diese Klemmgliedanordnungen gilt das zu den vorstehend an Hand der Fig. 1 bis 8 beschriebenen Klemmgliedanordnungen Gesagte.

In den Schnittansichten gemäß Fig. 9, Fig. 10 und Fig. 11 sind jeweils durch Strichpunktlinien markierten Bereiche angegeben, die Aufnahmeöffnungen 12, 13 darstellen sollen. Diese Aufnahmeöffnungen 12, 13 verlaufen im Unterschied zu den Aufnahmeöffnungen 12 gemäß Fig. 6 und Fig. 8 hier jeweils zwischen distalem Ende und proximalem Ende des jeweiligen Aufnahmekörpers 1, also in dessen Längsrichtung. Die betreffenden Öffnungen können jedoch auch jeweils quer zu Längsrichtung des Aufnahmekörpers 1 in diesem enthalten sein. In allen diesen Aufnahmeöffnungen, also nicht nur gemäß Fig. 9 bis 11, sondern auch gemäß Fig. 6 und 8, kann eine Lichtquelle untergebracht sein, die beispielsweise eine oder mehrere Leuchtdioden (LEDs) samt zugehöriger Stromquelle (zum Beispiel Batterien) umfassen kann. Der Aufnahmekörper 1 kann dabei aus einem lichtdurchlässigen Material bestehenden, so dass das von der betreffenden Lichtquelle abgegebene Licht den Aufnahmekörper 1 durchdringen und zu dessen Außenseite gelangen kann. Es ist aber auch möglich, die betreffende Lichtquelle auf der Außenseite des Aufnahmekörpers 1 anzuordnen, um Licht abzugeben. Wie eingangs erwähnt, kann eine solche Lichtabgabe für den mit einer Vorrichtung gemäß der Erfindung Hantierenden, wie einem Chirurgen, sehr hilfreich sein, um diese Vorrichtung nach deren Gebrauch in üblicherweise abgedunkelten Arbeitsräumen, wie Operationssälen wieder schnell auffinden zu können.

Alternativ oder zusätzlich zu der vorstehend erläuterten Maßnahme, in und/oder an dem Aufnahmekörper 1 eine Lichtquelle vorzusehen, kann gemäß der Erfindung der Aufnahmekörper 1 auf seiner Außenfläche zumindest partiell phosphoreszierend oder fluoreszierend ausgebildet sein. Dadurch kann eine Vorrichtung gemäß der Erfindung nach zuvor erfolgter Bestrahlung mit für die Entfaltung der Phosphoreszenz oder Fluoreszenz passender Strahlung in üblicher Weise abgedunkelten Arbeitsräumen, wie Operationssälen wieder schnell lokalisiert und somit erfasst werden, in denen mit einer solchen Vorrichtung und dem von dieser aufgenommenen Führungsdraht gearbeitet wird.

Wie im Zusammenhang mit Fig. 1 erwähnt, enthält die dort als erstes Ausführungsbeispiel gezeigte Vorrichtung gemäß der Erfindung einen zylinderförmigen Aufnahmekörper 1, der vorzugsweise aus einem biokompatiblen Material, wie Acrylnitril-Butadien-Styrol (ABS) besteht. Diese Materialwahl trifft auch für die Vorrichtungen gemäß den anderen vorstehend erläuterten Ausführungsbeispielen zu. Auch die im Zusammenhang mit dem ersten Ausführungsbeispiel erwähnte Vorgehensweise, nämlich jedes der dort zusammen mit dem Aufnahmekörper 1 geformten Klemmelemente 5 und 8 als gesondertes Klemmelement vorzusehen und mit dem Aufnahmekörper zu verbinden, kann auch bei allen anderen erläuterten Ausführungsbeispielen entsprechend angewandt werden. Dabei kann jedes derartige gesonderte Klemmelement gegebenenfalls aus einem anderen, vorzugsweise biokompatiblen Material, wie beispielsweise aus Polyoxymethylen (POM) bestehen als aus dem Material, aus dem der zugehörige Aufahmekörper 1 besteht.

**Bezugzeichenliste**

| | |
|---|---|
| 1 | Aufnahmekörper |
| 2 | Führungsdraht |
| 3 | Längsöffnung |
| 4 | Öffnungsbereich |
| 5 | Klemmelement |
| 5' | Klemmelement |
| 6 | Abschrägungsfase |
| 6' | Abschrägungsfase |
| 7 | Kantenteil |
| 7' | Kantenteil |
| 8 | Klemmelement |
| 9 | Zwischenraum |
| 9' | Zwischenraum |
| 10 | Zwischenraum |
| 11 | Zwischenraum |
| 11' | Zwischenraum |
| 12 | Aufnahmeöffnung |
| 13 | Aufnahmeöffnung |

## Patentansprüche

1. Vorrichtung zum Festklemmen eines medizinischen Führungsdrahtes (2), umfassend einen Aufnahmekörper (1) mit einer diesen durchziehenden Längsöffnung (3) für die Aufnahme des Führungsdrahtes (2) der in der Längsöffnung (3) verschiebbar und verdrehbar ist, und einen Klemmmechanismus (5, 8; 5'), durch dessen Wirksamwerden die Verschieb- und/oder Verdrehbarkeit des in der genannten Längsöffnung (3) aufgenommenen Führungsdrahtes (2) blockierbar ist,
**dadurch gekennzeichnet,**
• **dass** der Klemmmechanismus (5, 8; 5') entweder nur an einem Ende der Längsöffnung (3) des Aufnahmekörpers (1) oder an jedem Ende der Längsöffnung (3) des Aufnahmekörpers (1) gebildet ist,
• **dass** der jeweilige Klemmmechanismus (5, 8; 5')
- einen an seinem zugehörigen Ende des Aufnahmekörpers (1) quer zu der Längsöffnung (3) des Aufnahmekörpers (1) verlaufenden erweiterten Öffnungsbereich (4), der sich zu der Längsöffnung (3) über eine Strecke hin erstreckt, die kürzer ist als die Längsöffnung (3) lang ist,
- und eine Klemmgliedanordnung (5, 8; 5') aufweist,
• und **dass** die jeweilige Klemmgliedanordnung (5, 8; 5')
- zwischen ihrem erweiterten Öffnungsbereich (4) und der genannten Längsöffnung (3) mit dem Aufnahmekörper (1) elastisch ausbiegbar verbunden ist,
- sich parallel zu der Längsöffnung (3) über eine Distanz erstreckt, die kürzer ist als die genannte Strecke in Richtung der Längsöffnung (3) lang ist,
- und an einer von dem betreffenden Ende abgewandten, in dem Öffnungsbereich (4) liegenden Seite einen dem zugehörigen Öffnungsbereich (4) zugewandten Kantenteil (7; 7') aufweist, an dem der Führungsdraht (2) nach seinem Verschwenken aus der Längsöffnung (3) an dem betreffenden Ende des Aufnahmekörpers (1) und an der Klemmgliedanordnung (5, 8; 5') vorbei in den an diesem Ende des Aufnahmekörpers (1) vorhandenen Öffnungsbereich (4) hinein unter Blockierung seiner Verschieb- und/oder Verdrehbarkeit in Anlage bringbar ist.

2. Vorrichtung nach Anspruch 1,
**dadurch gekennzeichnet, dass** der jeweilige Öffnungsbereich (4) unter einem Winkel zwischen etwa 10° und 120° in Bezug auf die Längsöffnung des Aufnahmekörpers verläuft.

3. Vorrichtung nach Anspruch 1 oder 2,
**dadurch gekennzeichnet, dass** die jeweilige Klemmgliedanordnung (5') zwischen der Längsöffnung (3) und dem Öffnungsbereich (4) durch ein einziges Klemmelement (5') gebildet ist, welches mit einem Zwischenraum (9') zu einem Randbereich der Längsöffnung (3) vorgesehen ist, und dass der Zwischenraum (9') einen ein Hindurchdrücken des Führungsdrahtes (2) aus der Längsöffnung (3) in den genannten Öffnungsbereich (4) und von diesem in die Längsöffnung (3) ermöglichenden Abstand aufweist.

4. Vorrichtung nach einem der Ansprüche 1 bis 3,
**dadurch gekennzeichnet, dass** die jeweilige Klemmgliedanordnung (5, 8) zwischen der Längsöffnung (3) und ihrem zugehörigen Öffnungsbereich (4) durch zwei Klemmelemente (5, 8) gebildet ist, die unter Zurücklassen eines Zwischenraumes (9) zwischen ihnen jeweils an diametral gegenüber liegenden Stellen von Verbindungsbereichen zwischen der Längsöffnung (3) und dem betreffenden Öffnungsbereich (4) vorgesehen sind, und dass der Zwischenraum (9) einen ein Hindurchdrücken des Führungsdrahtes (2) aus der Längsöffnung (3) in den genannten Öffnungsbereich (4) und von diesem in die Längsöffnung (3) ermöglichenden Abstand aufweist.

5. Vorrichtung nach einem der Ansprüche 3 oder 4,
**dadurch gekennzeichnet, dass** das jeweilige Klemmelement (5, 8; 5') auf seiner der Längsöffnung (3) zugewandten Seite eine Abschrägungsfase (6; 6') aufweist.

6. Vorrichtung nach einem der Ansprüche 3 bis 5,
**dadurch gekennzeichnet, dass** das jeweilige Klemmelement (5, 8; 5') an dem Verbindungsbereich zwischen der Längsöffnung (3) und seinem zugehörigen Öffnungsbereich(4) an dem Aufnahmekörper (1) angeformt ist.

7. Vorrichtung nach einem der Ansprüche 3 bis 5,
**dadurch gekennzeichnet, dass** das jeweilige Klemmelement (5, 8; 5') aus einem vom Material des Aufnahmekörpers (1) verschiedenen Material besteht und an dem Verbindungsbereich zwischen der Längsöffnung (3) und seinem zugehörigen Öffnungsbereich (4) an dem Aufnahmekörper (1) gesondert befestigt ist.

8. Vorrichtung nach einem der Ansprüche 1 bis 7,
**dadurch gekennzeichnet, dass** sie aus einem Kunststoff, insbesondere biokompatiblen Kunststoff besteht.

9. Vorrichtung nach einem der Ansprüche 1 bis 8,
**dadurch gekennzeichnet, dass** der Aufnahmekörper (1) auf seiner Außenfläche zumindest partiell phosphoreszierend oder fluoreszierend ausgebildet ist.

10. Vorrichtung nach einem der Ansprüche 1 bis 9,
**dadurch gekennzeichnet, dass** sie wenigstens eine Lichtquelle, insbesondere in einer in dem aus einem lichtdurchlässigen Material bestehenden Aufnahmekörper (1) gebildeten Aufnahmeöffnung (12; 13) enthält.

## Claims

1. Device for clamping a medical guide wire (2), including a receiving body (1) with a longitudinal opening (3) traversing it for receiving the guide wire (2), which is displaceable and twistable in the longitudinal opening (3), and a clamping mechanism (5, 8; 5'), by the activation of which the displaceability and/or twistability of the guide wire (2) received in the mentioned longitudinal opening (3) can be blocked,
**characterized in that**
• the clamping mechanism (5, 8; 5') is formed either only at one end of the longitudinal opening (3) of the receiving body (1) or at each end of the longitudinal opening (3) of the receiving body (1),
• that the respective clamping mechanism (5, 8; 5') has
- an expanded opening area (4) extending transversely to the longitudinal opening (3) of the receiving body (1) at its associated end of the receiving body (1), which extends to the longitudinal opening (3) over a segment, which is shorter than the longitudinal opening (3) is long,
- and a clamping member assembly (5, 8; 5')
• and that the respective clamping member assembly (5, 8; 5')
- is connected to the receiving body (1) in elastically bendable manner between its expanded opening area (4) and the mentioned longitudinal opening (3),
- extends parallel to the longitudinal opening (3) over a distance, which is shorter than the mentioned segment is long in the direction of the longitudinal opening (3),
- and has an edge part (7, 7') facing the associated opening area (4) on a side facing away from the concerned end and located in the opening area (4), on which the guide wire (2) can be abutted blocking its displaceability and/or twistability after its swiveling from the longitudinal opening (3) past the concerned end of the receiving body (1) and the clamping member assembly (5, 8; 5') into the opening area (4) present at this end of the receiving body (1).

2. Device according to claim 1,
**characterized in that** the respective opening area (4) extends at an angle between about 10° and 120° with respect to the longitudinal opening of the receiving body.

3. Device according to claim 1 or 2,
**characterized in that** the respective clamping member assembly (5') is formed by a single clamping element (5') between the longitudinal opening (3) and the opening area (4), which is provided with a clearance (9') to a border area of the longitudinal opening (3), and that the clearance (9') has a distance allowing pressing-through the guide wire (2) from the longitudinal opening (3) into the mentioned opening area (4) and from it into the longitudinal opening (3).

4. Device according to any one of claims 1 to 3,
**characterized in that** the respective clamping member assembly (5, 8) is formed by two clamping elements (5, 8) between the longitudinal opening (3) and its associated opening area (4), which are respectively provided at diametrically opposing locations of connection areas between the longitudinal opening (3) and the concerned opening area (4) leaving a clearance (9) between them, and that the clearance (9) has a distance allowing pressing-through the guide wire (2) from the longitudinal opening (3) into the mentioned opening area (4) and from it into the longitudinal opening (3).

5. Device according to any one of claims 3 or 4,
**characterized in that** the respective clamping element (5, 8; 5') has a sloping chamfer (6; 6') on its side facing the longitudinal opening (3).

6. Device according to any one of claims 3 to 5,
**characterized in that** the respective clamping element (5, 8; 5') is formed at the receiving body (1) in the connection area between the longitudinal opening (3) and its associated opening area (4).

7. Device according to any one of claims 3 to 5,
**characterized in that** the respective clamping element (5, 8; 5') is composed of a material different from the material of the receiving body (1) and is separately fixed to the receiving body (1) in the connection area between the longitudinal opening (3) and its associated opening area (4).

8. Device according to any one of claims 1 to 7,
**characterized in that** it is composed of a plastic, in particular biocompatible plastic.

9. Device according to any one of claims 1 to 8,
**characterized in that** the receiving body (1) is at least partially phosphorescing or fluorescing formed on its exterior surface.

10. Device according to any one of claims 1 to 9,
**characterized in that** it contains at least one light source, in particular in a receiving opening (12; 13) formed in the receiving body (1) composed of a light-transmissive material.

## Revendications

1. Appareil, destiné à immobiliser un fil de guidage médical (2), comprenant un corps récepteur (1) avec une ouverture longitudinale (3), faisant passer celui-ci, pour le logement du fil de guidage (2), qui est susceptible d'être déplacé et de tourner dans l'ouverture longitudinale (3) et un mécanisme de serrage (5, 8 ; 5'), par la prise d'effet duquel la faculté de déplacement et / ou de rotation du fil de guidage (2), logé dans l'ouverture longitudinale (3) nommée, peut être bloquée,
**caractérisé en ce**
• **que** le mécanisme de serrage (5, 8 ; 5') est formé soit à une seule extrémité de l'ouverture longitudinale (3) du corps récepteur (1), soit à chaque extrémité de l'ouverture longitudinale (3) du corps récepteur (1),
• **que** le mécanisme de serrage (5, 8 ; 5') respectif présente
- une zone d'ouverture élargie (4), évoluant contre son extrémité associée du corps récepteur (1), transversalement par rapport à l'ouverture longitudinale (3) du corps récepteur (1), qui s'étend, par rapport à l'ouverture longitudinale (3), au-delà d'une section, qui est plus courte que l'ouverture longitudinale (3) n'est longue et
- un agencement formant organe de serrage (5, 8 ; 5') et
• **que** l'agencement formant organe de serrage (5, 8 ; 5') respectif
- est relié élastiquement, de manière à pouvoir être courbé, entre sa zone d'ouverture élargie (4) et l'ouverture longitudinale (3) nommée, au corps récepteur (1),
- s'étend parallèlement à l'ouverture longitudinale (3), sur une distance qui est plus courte que la section nommée n'est longue dans le sens de l'ouverture longitudinale (3) et
- présente, sur un côté, situé dans la zone d'ouverture (4), éloignée de l'extrémité concernée, une partie de bord (7 ; 7'), tournée vers la zone d'ouverture (4) associée, partie de bord (7 ; 7') contre laquelle peut être amené en appui le fil de guidage (2), après son pivotement depuis l'ouverture longitudinale (3) contre l'extrémité concernée du corps récepteur (1) et contre l'agencement formant organe de serrage (5, 8 ; 5'), dans la zone d'ouverture (4), présente à cette extrémité du corps récepteur (1), avec blocage de sa faculté de déplacement et / ou de rotation.

2. Appareil selon la revendication 1,
**caractérisé en ce que**
la zone d'ouverture (4) respective évolue suivant un angle entre environ 10° et 120° par rapport à l'ouverture longitudinale du corps récepteur.

3. Appareil selon la revendication 1 ou 2,
**caractérisé en ce que**
l'agencement formant organe de serrage (5') respectif est formé, entre l'ouverture longitudinale (3) et zone d'ouverture (4), par un seul élément de serrage (5'), lequel est prévu avec un espace intermédiaire (9') par rapport à une zone de bordure de l'ouverture longitudinale (3) et que l'espace intermédiaire (9') présente un intervalle, permettant une insertion en force du fil de guidage (2) à partir de l'ouverture longitudinale (3) dans la zone d'ouverture (4) nommée et, à partir de là, dans l'ouverture longitudinale (3).

4. Appareil selon l'une des revendications 1 à 3,
**caractérisé en ce que**
l'agencement formant organe de serrage (5, 8) respectif est formé, entre l'ouverture longitudinale (3) et sa zone d'ouverture (4) associée, par deux éléments de serrage (5, 8), qui sont prévus, avec abandon d'un espace intermédiaire (9) entre eux, respectivement, à des endroits, situés diamétralement opposés, de zones de liaison entre l'ouverture longitudinale (3) et la zone d'ouverture (4) concernée et que l'espace intermédiaire (9) présente un intervalle, permettant une insertion en force du fil de guidage (2) depuis l'ouverture longitudinale (3) dans la zone d'ouverture (4) nommée et, à partir de là, dans l'ouverture longitudinale (3).

5. Appareil selon l'une des revendications 3 ou 4,
**caractérisé en ce que**
l'élément de serrage (5, 8 ; 5') respectif présente, sur son côté tourné vers l'ouverture longitudinale (3), une facette formant évasement (6 ; 6').

6. Appareil selon l'une des revendications 3 à 5,
**caractérisé en ce que**
l'élément de serrage (5, 8 ; 5') respectif est formé contre la zone de liaison entre l'ouverture longitudinale (3) et sa zone d'ouverture (4) associée contre le corps récepteur (1).

7. Appareil selon l'une des revendications 3 à 5,
**caractérisé en ce que**
l'élément de serrage (5, 8 ; 5') respectif se compose d'un matériau différent du matériau du corps récepteur (1) et est fixé séparément contre la zone de liaison entre l'ouverture longitudinale (3) et sa zone d'ouverture (4) associée contre le corps récepteur (1).

8. Appareil selon l'une des revendications 1 à 7,
**caractérisé en ce**
**qu'**il se compose d'un plastique, en particulier d'un plastique biocompatible.

9. Appareil selon l'une des revendications 1 à 8,
**caractérisé en ce que**
le corps récepteur (1) est constitué sur sa surface extérieure au moins partiellement de manière phosphorescente ou fluorescente.

10. Appareil selon l'une des revendications 1 à 9,
**caractérisé en ce**
**qu'**il contient au moins une source lumineuse, en particulier dans une ouverture réceptrice (12 ; 13), formée dans le corps récepteur (1), se composant d'un matériau transparent.
